(19) Europäisches Patentamt
European Patent Office
Office européen des brevets

(11) Veröffentlichungsnummer: **0 262 498 B1**

# (12) EUROPÄISCHE PATENTSCHRIFT

(45) Veröffentlichungstag der Patentschrift: **30.05.90**

(51) Int. Cl.⁵: **C07F 15/00**, A61K 31/28, C07D 209/12

(21) Anmeldenummer: **87113384.9**

(22) Anmeldetag: **14.09.87**

(54) **Diamin-platin (II)-Komplexverbindungen mit einem hydroxylierten 2-Phenyl-Indolring.**

(30) Priorität: **03.10.86 DE 3633673**

(43) Veröffentlichungstag der Anmeldung: **06.04.88 Patentblatt 88/14**

(45) Bekanntmachung des Hinweises auf die Patenterteilung: **30.05.90 Patentblatt 90/22**

(84) Benannte Vertragsstaaten: **AT BE CH DE ES FR GB GR IT LI LU NL SE**

(56) Entgegenhaltungen:
**EP-A- 0 193 083**
**FR-A- 2 512 466**

(73) Patentinhaber: **ASTA Pharma Aktiengesellschaft, Weismüllerstrasse 45, D-6000 Frankfurt am Main 1(DE)**

(72) Erfinder: **von Angerer, Erwin, Dr., Regensburger Strasse 6, D-8401 Grasslfing(DE)**
Erfinder: **Knebel, Norbert, Härtinger Strasse 4, D-8070 Ingolstadt(DE)**
Erfinder: **Schönenberger, Helmut, Prof. Dr, Ahornstrasse 14, D-8401 Pentling(DE)**
Erfinder: **Engel, Jürgen, Dr., Erlenweg 3, D-8755 Alzenau(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 262 498 B1

**Beschreibung**

Die britische Patentschrift 2 111 478 betrifft tumorhemmende Indol-Derivate der folgenden Formel

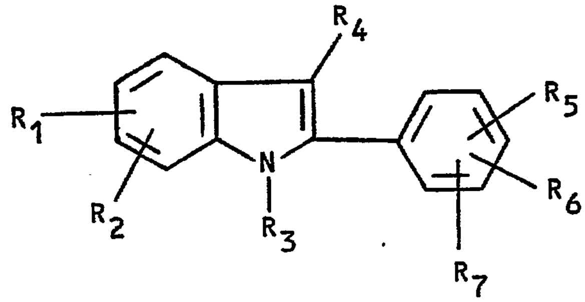

worin $R_1$ Wasserstoff, eine Hydroxygruppe oder eine $C_2$–$C_6$-Alkanoyloxygruppe, $R_2$ Wasserstoff, eine Hydroxygruppe, eine $C_2$–$C_6$-Alkanoyloxygruppe oder ein Halogenatom, $R_3$ eine $C_1$–$C_6$-Alkylgruppe, $R_4$ Wasserstoff oder eine $C_1$–$C_6$-Alkylgruppe, $R_5$ eine Hydroxygruppe oder eine $C_2$–$C_6$-Alkanoyloxygruppe, $R_6$ Wasserstoff oder ein Halogenatom und $R_7$ Wasserstoff oder ein Halogenatom bedeutet.

Weiterhin sind tumorhemmende Ethylendiamin-platin(II)Komplexe bekannt, worin eine $CH_2$-Gruppe des Ethylendiamins durch einen Benzylrest, Phenylethylrest, Thienylmethylrest, Indolylmethylrest oder Imidazolylmethylrest substituiert ist (DE-OS 3 605 191), oder worin beide $CH_2$-Gruppen des Ethylendiamins einen Phenylrest beziehungsweise einen substituierten Phenylrest enthalten (DE-OS 3 405 611; DE-OS 3 604 866).

Die Erfindung betrifft neue cis-Diamin-platin(II)-Komplexverbindungen mit einem hydroxylierten 2-Phenyl-Indolring, die als Arzneimittelwirkstoffe verwendbar sind.

Es handelt sich um cis-Platin-Komplexe der allgemeinen Formel

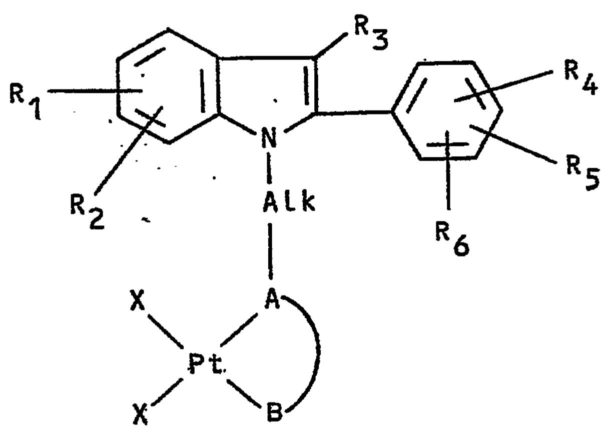

I

worin $R_1$ Wasserstoff, eine Hydroxygruppe oder eine $C_2$–$C_6$-Alkanoyloxygruppe, $R_2$ Wasserstoff oder ein Halogenatom, $R_3$ Wasserstoff oder eine $C_1$–$C_6$-Alkylgruppe, $R_4$ eine Hydroxygruppe oder eine $C_2$–$C_6$-Alkanoyloxygruppe, $R_5$ Wasserstoff oder ein Halogenatom und $R_6$ Wasserstoff oder ein Halogenatom bedeutet, Alk eine $C_2$–$C_{10}$-Alkylenkette darstellt, wobei 4 benachbarte $CH_2$-Gruppen auch durch einen 1,4-Phenylenring ersetzt sein können, A–B die Gruppe HN–$CH_2$–$CH_2$–$NH_2$, $H_2N$–$CH_2$–CH–$CH_2$–$NH_2$ oder

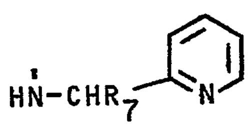

bedeutet, und $R_7$ ein Wasserstoffatom, eine $C_1$–$C_6$-Alkylgruppe oder ein Phenylrest ist und X für das Äquivalent eines physiologisch verträglichen Anions steht.

Das Herstellungsverfahren ist dadurch gekennzeichnet, daß man eine Tetrahalogeno-platin(II)-säure, ein Tetrahalogeno-platin(II)-Komplexsalz mit zwei einwertigen oder einem zweiwertigen Kation oder

ein Platin(II)-halogenid mit einer Verbindung der Formel

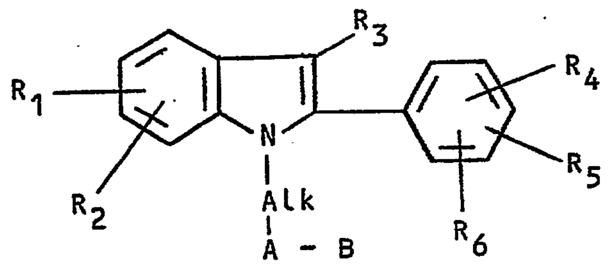

II

oder einem Salz der Verbindung II umsetzt, wobei die Reste $R_1$, $R_2$, $R_3$, $R_4$, $R_5$, $R_6$ und die Gruppen Alk und A–B die angegebenen Bedeutungen haben und gegebenenfalls in einer erhaltenen Verbindung der Formel I den Rest X beziehungsweise die Reste X gegen andere physiologisch verträgliche Anionen austauscht.

Die neuen erfindungsgemäßen Verbindungen besitzen eine ausgeprägte Antitumorwirkung bei guter Verträglichkeit. Die Wirkung zeigt sich insbesondere bei folgenden Tier- und Zellkulturmodellen: Transplantiertes hormonabhängiges MXT-Mammacarcinom der BDF-1-Maus- östrogenabhängiges dimethylbenzanthraceninduziertes Mammacarcinom der SD-Ratte. Menschliche hormonabhängige MCF-7 Brustkrebszellen.

Der überraschende erfinderische Fortschritt der erfindungsgemäßen Verbindungen liegt darin, daß diese Platinkomplexe darstellen, die eine sehr hohe Affinität zum Östronrezeptor aufweisen und eine selektive Wirkung an östrogenrezeptorhaltigen Tumoren wie dem Mammacarcinom entfalten. Es ist damit erstmals gelungen, ein östrogenrezeptoraffines Molekül mit einer cytostatisch wirksamen Diaminplatingruppierung so zu verknüpfen, daß die Affinität zum Rezeptor und die cytostatische Wirkung erhalten bleiben.

Die erfindungsgemäßen Platinkomplexe besitzen zum Beispiel eine hohe Bindungsaffinität zum Östrogenrezeptor aus Kalbsuteri. Die relativen Bindungsaffinitäten liegen in der Größenordnung von 1–15% der Affinität des Östradiols. Trotz der starken Bindung an den Östrogenrezeptor zeigen die Komplexe am Uterus der Maus jedoch keine östrogene Wirkung. In einigen Fällen konnte sogar eine deutliche antiöstrogene Wirkung nachgewiesen werden (Verbindungen gemäß den Beispielen 2 und 5). Am transplantierten hormonabhängigen MXT-Mammacarcinom der Maus wurde eine starke tumorhemmende Wirkung gefunden. So beträgt zum Beispiel die Hemmwirkung der Verbindung gemäß Beispiel 1 bei einer Dosis von 20 mg/kg 89 % nach 6 Wochen Behandlung. Die Hemmwirkung der Verbindung gemäß Beispiel 2 unter den gleichen Bedingungen ist 77%.

Die folgenden Angaben betreffen Ausgestaltungen der Erfindung hinsichtlich der Substituenten R, der Alkylenbrücke Alk, der Gruppe A – B sowie der Säureanionen X.

Die in den einzelnen Resten $R_1$ bis $R_7$ vorkommenden $C_1$–$C_6$-Alkylgruppen, $C_2$–$C_6$-Alkanoyloxygruppen sowie die Alkylenbrücke Alk können gerade oder verzweigt sein. Die Alkylgruppen bestehen vorzugsweise aus 1, 2, 3 oder 4 C-Atomen, die Alkanoyloxygruppen vorzugsweise aus 2 – 4 C-Atomen (insbesondere handelt es sich hier um die Acetoxygruppe). Der Rest $R_3$ ist vorzugsweise Methyl, der Rest $R_7$ vorzugsweise Wasserstoff, Methyl, Ethyl oder Phenyl. Der Rest $R_1$ befindet sich vorzugsweise in der 5– oder 6-Stellung des Indolringes. Der Rest $R_4$ befindet sich vorzugsweise in der 4-Stellung des Phenylringes, die Reste $R_5$ und $R_6$ vorzugsweise in 2– und/oder 6-Stellung.

Die Alkylenbrücke Alk besteht beispielsweise aus 4 bis 8 $CH_2$-Gruppen, vorzugsweise 5, 6 oder 7 $CH_2$-Gruppen. Falls 4 benachbarte $CH_2$-Gruppen von Alk durch einen 1,4-Phenylenring ersetzt sind, kann es sich hierbei um 4 benachbarte $CH_2$-Gruppen handeln, die nicht an ein N-Atom gebunden sind, oder der Phenylenring kann auch direkt an ein N-Atom oder mit beiden N-Atomen verknüpft sein, wobei im letzteren Falle die Brücke Alk den 1,4-Phenylenring darstellt.

Der Rest $R_2$ befindet sich beispielsweise in der 4-Stellung des Indolringes.

Weitere Beispiele für die Gruppe Alk mit einem Phenylenring sind:

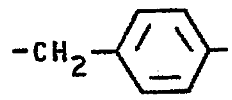

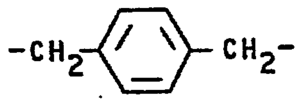

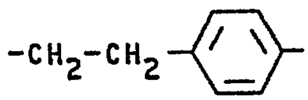

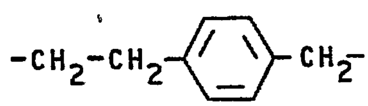

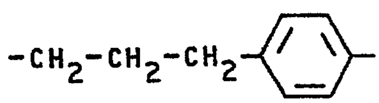

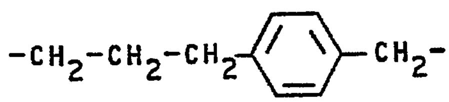

Als Halogensubstituenten kommen insbesondere Chlor, Brom und/oder Fluor in Frage.

Die Reste X stellen die bekannten und üblichen physiologisch verträglichen und pharmazeutisch verwendbaren Anionen ein- oder mehrwertiger Säuren dar. Insbesondere kommen beispielsweise die Anionen folgender Säuren in Frage: HBr, HCl, HJ, HF, $HNO_3$, $H_2SO_4$ ($SO_4^{--}$); $H_3PO_4$ ($HPO_4^{--}$); $H_2CO_3$ ($CO_3^{--}$);Kampfersulfonsäure, aliphatische oder aromatische Sulfonsäuren, beispielsweise $C_1$–$C_6$-Alkylsulfonsäuren (zum Beispiel Methansulfonsäure, Ethan-, Propan- oder Hexansulfonsäure, Benzol- oder Naphthalinsulfonsäure, die gegebenenfalls ein- oder zweifach durch Methylgruppen substituiert sind (Toluolsulfonsäure, insbesondere o– oder p-Toluolsulfonsäure); aliphatische $C_1$–$C_4$-Monocarbonsäuren, die gegebenenfalls ein-, zwei- oder dreifach durch Halogenatome (insbesondere Cl, F) substituiert sind (zum Beispiel Ameisensäure, Essigsäure, Propionsäure, Chloressigsäure, Dichloressigsäure, Trifluoressigsäure, Trichloressigsäure); aliphatische $C_2$–$C_{11}$Dicarbonsäuren, die gegebenenfalls eine Doppelbindung enthalten (zum Beispiel Oxalsäure, Malonsäure, 2-Amino-Malonsäure, Malonsäure, welche in 2-Stellung durch eine Benzylgruppe oder eine oder zwei $C_1$–$C_4$-Alkylgruppen substituiert ist, Maleinsäure, Fumarsäure, Bernsteinsäure); aliphatische Monohydroxy- und Dihydroxy-Monocarbonsäuren mit 2 bis 6, insbesondere 2 bis 3 Kohlenstoffatomen, wobei es sich vorzugsweise um α-Monohydroxycarbonsäuren handelt wie Milchsäure, Glycerinsäure oder Glykolsäure; aliphatische Monohydroxy- und Dihydroxy-, Di- und Tricarbonsäuren mit 3 bis 8 Kohlenstoffatomen, insbesondere 3 bis 6 Kohlenstoffatomen wie Äpfelsäure, Weinsäure, Malonsäure, die an dem mittelständigen C-Atom durch eine Hydroxygruppe und gegebenenfalls eine $C_1$–$C_4$-Alkylgruppe substituiert sein kann, Isozitronensäure oder Zitronensäure; Phthalsäure, die gebenenfalls durch eine Carboxygruppe (insbesondere in 4-Stellung) substituiert ist; Gluconsäure; Glucuronsäure; die natürlichen α-Aminosäuren (zum BeispieL L-Asparaginsäure); 1,1-Cyclobutandicarbonsäure; Organophosphorsäuren, wie Aldose und Ketosephosphorsäuren (beispielsweise die entsprechenden Mono- und Diphosphorsäuren) zum Beispiel Aldose-6-phosphorsäuren wie D- oder L-Glucose-6-phosphorsäure, α-D-Glucose-1-phosphorsäure, D-Fructose-6-phosphorsäure, D-Galactose-6-phosphor-säure, D-Ribose-5-phosphorsäure, D-Fructose-1,6-diphosphorsäuren; Glycerinphosphorsäuren (wobei der Phosphorsäurerest an einem der endständigen oder an dem mittelständigen Glycerinsauerstoffatom gebunden ist) wie α-D, L-Glycerin-phosphorsäure, β-Glycerinphosphorsäure; N-Phosphono-acetyl-Asparaginsäure.

Ebenfalls kommen als Säuren, die die Anionen X bilden, Aminosäuren beziehungsweise Aminosäurederivate, deren basische Aminogruppe durch eine Säuregruppe neutralisiert ist, in Frage. Es handelt sich hierbei zum Beispiel um Aminosäuren der folgenden Struktur:

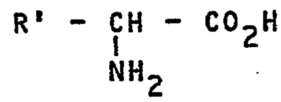

worin R′ Wasserstoff, einen Phenylrest, einen Indolyl-(3)-methylrest, Imidazolyl-(4)-methylrest, eine $C_1$–$C_{10}$-Alkylgruppe oder eine $C_1$–$C_{10}$-Alkylgruppe, die durch eine Hydroxygruppe, eine Carboxygruppe, eine $C_1$–$C_6$-Alkoxygruppe, eine Mercaptogruppe, eine $C_1$–$C_6$-Alkylthiogruppe, eine Phenylgruppe, eine Hydroxyphenylgruppe, eine $C_2$–$C_6$-Alkanoylaminogruppe oder eine $C_1$–$C_6$-Alkoxycarbonylgruppe substituiert ist, bedeutet.

Die basische Aminogruppe in 2-Stellung ist hierbei durch eine übliche Aminosäureschutzgruppe neutralisiert (acyliert), beispielsweise durch einen $C_2$–$C_6$-Alkanolrest oder den Butyloxycarbonylrest.

Falls in der obigen Formel R′ eine Alkylgruppe ist, handelt es sich vorzugsweise um eine $C_1$–$C_6$-Alkylgruppe, die zum Beispiel in 2-, 3-, 4-, 5- oder 6-Stellung (Zählung beginnt an der Verknüpfungsstelle des Alkylrestes mit dem Restmolekül) eine $C_2$–$C_6$-Alkanoylaminogruppe, einen Imidazolyl-(4)-methylrest oder einen Indolyl-(3)-methylrest enthält. Einzelbeispiele für solche Aminosäuren sind: Leucin (vorzugsweise D- und L-Form), Valin (vorzugsweise D- und L-Form), Phenylalanin (vorzugsweise D- und L-Form), Phenylglycin (vorzugsweise D- und L-Form), Alanin (vorzugsweise D- und L-Form), Isoleucin (vorzugsweise D- und L-Form), Asparagin (vorzugsweise D- und L-Form), Lysin (vorzugsweise D- und L-Form), Tryptophan (vorzugsweise D- und L-Form), Tyrosin (vorzugsweise D- und L-Form), Ornithin (vorzugsweise D- und L-Form).

Hierbei sind die basischen Aminogruppen durch eine übliche Acylaminoschutzgruppe blockiert, insbesondere durch die Acetylgruppe oder die Butyloxycarbonylgruppe.

Die Formel I umfaßt auch die möglichen Enantiomere und Diastereomere. Falls die Verbindungen Racemate sind, können diese in an sich bekannter Weise, zum Beispiel mittels einer optisch aktiven Säure in die optisch aktiven Isomere gespalten werden. Es ist aber auch möglich, von vornherein enantiomere oder gegebenenfalls auch diastereomere Ausgangsstoffe einzusetzen, wobei dann als Endprodukt eine entsprechende reine optisch aktive beziehungsweise diastereomere Verbindung erhalten wird. Zusätzliche Formen können durch verschiedene enantiomere beziehungsweise diastereomere Formen der Reste X entstehen.

Die Verbindungen der Formel I können gegebenenfalls Wasser enthalten und können dann auch als Diaquokomplexe vorliegen, wo 2 Moleküle Wasser komplex an das Platin gebunden sind; das Anion X beziehungsweise die Anionen X neutralisieren auch in diesen Fällen die 2fache positive Ladung des $Pt^{++}$-Kations.

Hinsichtlich des Platinatoms handelt es sich bei den erfindungsgemäßen Verbindungen der Formel I stets um die cis-Verbindungen.

Das Ausgangsamin II wird beispielsweise als Racemat, als reine rechts- beziehungsweise linksdrehende Form oder in einer sonstigen diastereomeren Form eingesetzt.

Diese Konfiguration bleibt bei der Herstellung des Platinkomplexes erhalten.

Das Verfahren zur Herstellung der erfindungsgemäßen Verbindungen I wird in einem Lösungsmittel oder Suspensionsmittel bei Temperaturen zwischen 10 und 80°C, vorzugsweise 20 bis 50°C, insbesondere 35 bis 45°C durchgeführt. Als Lösungsmittel kommen beispielsweise in Frage:
Wasser, $C_1$–$C_6$-Alkanole (Methanol, Ethanol, tert.-Butanol), Tetrahydrofuran, Dioxan, niedere Dialkylsulfoxide (zum Beispiel Dimethylsulfoxid), $C_1$–$C_4$-Mono- oder Dialkylamide von $C_1$–$C_4$-Alkancarbonsäuren (zum Beispiel Dimethylformamid), Ethylenglykoldimethylether, Diethylenglykoldimethylether sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 5,5 und 8, vorzugsweise bei pH 7 liegen. Die Einstellung des pH-Wertes erfolgt insbesondere durch Zusatz von Alkali, vorzugsweise wäßriger Natronlauge oder Kalilauge oder beispielsweise auch mittels Natriumcarbonat beziehungsweise durch Zusatz von Säuren, vorzugsweise wäßriger Salzsäure.

Als Tetrahalogen-platin(II)-Verbindungen (Säure sowie Komplexsalze) kommen die entsprechenden Tetrachloro-, Tetrabromo- und Tetrajodo-Verbindungen in Frage. Falls Platin(II)-halogenide als Ausgangskomponente eingesetzt werden, kommen die gleichen Halogenatome in Frage.

Als einwertige Kationen kommen in Betracht: Alkali-Ionen, insbesondere Natrium und Kalium; es können aber auch Lithium, Rubidium, Cäsium verwendet werden, ebenso $NH_4^+$, $NR_4^+$, $PR_4^+$ oder $AsR_4^+$, in denen R ein $C_1$–$C_6$-Alkylrest oder ein Phenylrest ist. Zweiwertige Kationen können sein: Erdalkali-Ionen, insbesondere $Mg^{2+}$ und $Ca^{2+}$, aber auch $Zn^{2+}$. Als Platin(II)-halogenide kommen beispielsweise $PtCl_2$, $PtBr_2$ und $PtJ_2$ in Frage.

Die Verbindung II wird entweder in Form des Diamins oder in Form eines Säureadditionssalzes eingesetzt:
zum Beispiel als Monohydrochlorid oder Dihydrochlorid, Mono- oder Dihydrobromid, Mono- oder Dihydrojodid oder als Salz mit einer anderen üblichen Säure, wie Schwefelsäure, Salpetersäure, Perchlorsäure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II in Form des Carbonats eingesetzt werden.

Der Austausch der Liganden X gegen andere Liganden kann beispielsweise mittels der Silberhalogenidfällung erfolgen. Hierzu wird beispielsweise eine Dihalogeno-diamino-platin(II)-Verbindung der Formel I, worin X Halogen (Chlor, Brom oder Jod) bedeutet in einem Lösungs- oder Suspensionsmittel bei Temperaturen zwischen 0 bis 90°C, vorzugsweise 10 bis 50°C, insbesondere 30 bis 40°C, vorzugsweise 40°C mit den Silbersalzen einer anderen Säure, die der Bedeutung X entspricht, umgesetzt. Man kann hierbei aber auch als Silbersalz Silbernitrat (zum Beispiel wäßrige Silbernitrat-Lösung) verwenden und erhält einen ionischen Diaquokomplex der Formel I, wobei jedes X ein Wassermolekül darstellt, der dann 2fach positiv geladen ist, und durch 2 $NO_3^-$-Anionen neutralisiert wird.

Aus diesem Komplex läßt sich der schwach gebundene Ligand Wasser leicht durch affinere Anionen (zum Beispiel $Cl^-$, $Br^-$) in Form von NaCl, KCl, NaBr, KBr, Malonat$^{2-}$, Chloracetat$^{(-)}$, Oxalat$^{2-}$, 1,1-Cyclobutandicarbonsäure-Anion$^{2-}$ sowie die übrigen angegebenen Säurereste X verdrängen, angewandt in Form der Säuren oder ihrer Salze, insbesondere ihrer Alkalisalze.

Die gleichen Verbindungen lassen sich auch durch Umsetzung äquimolarer Mengen von HX und Nitrat-freiem Platinkomplex (letzterer unter Verwendung von Anionenaustauschern in der Hydroxidform, zum Beispiel Dowex® 1 – 8X) gewinnen.

Ein Austausch der Abgangsgruppe (zum Beispiel $SO_4^{2-}$-beziehungsweise Oxalatanion$^{2-}$) ist im Falle der Sulfato- beziehungsweise Oxalato-diaminoplatin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt.

Für dieses Verfahren geeignete X-Liganden sind vorzugsweise die Anionen von Hydroxycarbonsäuren, Sulfonsäuren, Halogenessigsäuren, Salpetersäure.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser und Dimethylformamid sowie ferner Methanol, Ethanol, tert.-Butanol). Die Austauschreaktion wird beispielsweise in einem pH-Bereich zwischen 3 und 7 durchgeführt.

Die Herstellung von nichtbekannten Ausgangssubstanzen der Formel II kann zum Beispiel so erfolgen wie bei den einzelnen Beispielen angegeben ist, beziehungsweise analog hierzu.

Beispielsweise kann die Herstellung solcher Ausgangsverbindungen auf folgende Weise erfolgen:
Die entsprechende Indolverbindung mit den angegebenen Bedeutungen von $R_1$ bis $R_6$ (Hydroxygruppen sind zweckmäßig veräthert, zum Beispiel methyliert) wird in üblicher Weise mittels NaH oder einer äquivalenten Alkaliverbindung/Alkalimetall in das Derivat überführt, wo der Wasserstoff des Indolstickstoffs durch ein Alkalimetall ersetzt ist. Dieses Indol-Derivat wird dann in einem Lösungs- oder Suspensionsmittel bei Temperaturen zwischen –70 und +80°C mit einer Verbindung Hal-Alk-Hal (Hal zum Beispiel Brom) umgesetzt; die so erhaltene Verbindung, welche am Indolstickstoff die Gruppe Alk-Hal enthält, wird nun in einem Lösungs- oder Suspensionsmittel (zum Beispiel niedere Alkohole, niedere Dialkylether oder auch Amin HA-B) bei Temperaturen zwischen 10 und 150°C mit einem Amin HA-B umgesetzt. Zum Schluß werden gegebenenfalls vorhandene Ethergruppen (Methylgruppen) in üblicher Weise abgespalten (zum Beispiel mittels $BBr_3$ in einem halogenierten Kohlenwasserstoff bei 0 bis 50°C). Anstelle der Umsetzung mit dem Amin HA-B kann das Halogenatom der Gruppe Alk-Hal auch durch die $NH_2$-Gruppe ersetzt werden (zum Beispiel durch übliche Reaktion mit Phthalimidkalium in einem organischen Dialkylamid bei Temperaturen zwischen 50 und 180°C). Die Einführung der Gruppe A-B erfolgt dann durch Umsetzung mit einem entsprechenden Aldehyd oder Keton (der Strukturteil $H_2N-CH_2$ beziehungsweise $H_2N-CHR_7$ von HA-B ist dann die Gruppe CH=O beziehungsweise $CR_7$=O).

Diese Umsetzung erfolgt zum Beispiel in üblicher Weise in einem inerten Lösungsmittel zwischen 70 und 150°C. Anschließend wird in üblicher Weise die Doppelbindung der so erhaltenen Schiff'schen Base reduziert und eventuell vorhandene Ethergruppen werden abgespalten.

Wenn die Gruppe A-B die Bedeutung $H_2N-CH_2-CH-CH_2-NH_2$ hat, erfolgt die Herstellung der entsprechenden Ausgangssubstanzen zum Beispiel wie folgt:
Übliche Umsetzung von Malonsäuredinitril in Gegenwart von NaH mit dem entsprechenden Indol-Derivat, wo der Indolstickstoff die Gruppe AlkHal enthält, in einem inerten Mittel bei Temperaturen zwischen

0 und 80°C, Reduktion der beiden Nitrilgruppen zu Aminogruppen (zum Beispiel mittels komplexen Alkalihydriden des Aluminiums oder Bors bei Temperaturen zwischen 0 und 150°C) und gegebenenfalls anschließende Abspaltung von Ethergruppen.

In Ausgangsstoffen der Formel II beziehungsweise deren Vorstufen können vorhandene Hydroxygruppen durch $C_2$–$C_6$-Alkanoylgruppen acyliert werden.

Diese Acylierung kann beispielsweise mittels $C_2$–$C_6$-Alkanoylhalogeniden oder den Anhydriden der gesättigten aliphatischen $C_2$–$C_6$-Monocarbonsäuren bei Temperaturen zwischen 10 und 80°C, insbesondere 20 bis 30°C in Anwesenheit üblicher säurebindender Stoffe erfolgen. Insbesondere kommen als säurebindende Stoffe aliphatische tertiäre Amine, wie zum Beispiel Diisopropylethylamin in Betracht. Als inerte Lösungs- beziehungsweise Suspensionsmittel für die Acylierung kommen beispielsweise in Frage: niedere aliphatische Halogenkohlenwasserstoffe (Chloroform), aprotische Lösungsmittel wie Amide, $C_1$–$C_4$-Alkylamide und $C_1$–$C_4$-Dialkylamide aliphatischer $C_1$–$C_4$-Carbonsäuren (Dimethylformamid, Dimethylacetamid), N-Methyl-pyrrolidon, Dimethylsulfoxid oder Mischungen dieser Mittel.

Man kann diese Acylierung zum Beispiel aber auch in einem Zweiphasensystem beispielsweise Wasser/Chloroform durchführen, wobei der unter Zuhilfenahme eines Anionenaustauschers gewonnene acylierte Platin(II)-Komplex sich unlöslich abscheidet und das Gemisch von Säurechlorid und tertiärem Amin (Diisopropylethylamin) sich in der Chloroformphase befindet. Als Säurehalogenide kommen vorzugsweise die entsprechenden Chloride, Bromide und gegebenenfalls Jodide in Betracht. Als Anhydride kommen in Frage: Benzoesäureanhydrid sowie die Anhydride von $C_1$–$C_6$-Carbonsäuren, zum Beispiel symmetrische Säureanhydride wie Acetanhydrid, Propionsäureanhydrid, Buttersäureanhydrid.

Gegebenenfalls ist es unter Umständen zweckmäßig, vor einer solchen Acylierung vorhandene freie Aminogruppen mit abspaltbaren Schutzgruppen zu versehen. Es kommen hierbei die in der Peptidsynthese üblichen Schutzgruppen in Frage (zum Beispiel tert.-Butyloxycarbonylgruppe, 2-Nitro-phenylsulfenylgruppe). Nach der Acylierung werden diese Schutzgruppen in bekannter Weise abgespalten.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen oder auch Mischungen derselben mit anderen pharmazeutisch wirksamen Stoffen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, subkutan) oder oral angewendet werden. Beispielsweise kann die Verabreichung in Form von Tabletten, Kapseln, Pillen, Dragees oder Zäpfchen erfolgen. Als Liquida kommen zum Beispiel in Frage: ölige oder wäßrige Lösungen oder Suspensionen (zum Beispiel in Sesam- oder Olivenöl), Emulsionen, injizierbare wäßrige und ölige Lösungen oder Suspensionen. Weiterhin können beispielsweise Trockenampullen, welche als Wirkstoff die erfindungsgemäße Verbindung I enthalten, hergestellt werden, wobei vor Gebrauch der Inhalt solcher Trockenampullen zum Beispiel in physiologischer Kochsalzlösung oder Gemischen aus physiologischer Kochsalzlösung und beispielsweise flüssigen Polyethylenglykolen aufgelöst wird.

Die erfindungsgemäßen Verbindungen zeigen zum Beispiel am transplantierbaren hormonabhängigen MXT-Mammacarcinom der BDF-1-Maus beziehungsweise Ratte eine gute tumorhemmende Wirkung.

Beispielsweise wird an diesem Versuchsmodell bei einer Dosis von 20 mg/kg Körpergewicht bei der Maus das Tumorgewicht gegenüber der Kontrolle stark (zum Beispiel um 89%) verringert.

Die niedrigste, bereits wirksame Dosis in dem oben angegebenen Tierversuch ist beispielsweise 10 mg/kg subcutan.

Als allgemeiner Dosisbereich für die Wirkung (Tierversuch wie oben) kommt beispielsweise in Frage: 1 – 50 mg/kg subcutan, insbesondere 5 – 30 mg.

Die Wirkungsrichtung der erfindungsgemäßen Verbindungen ist mit der Wirkung des bekannten Arzneimittelwirkstoffs Estramustinphosphat vergleichbar; jedoch besitzen die erfindungsgemäßen Verbindungen im Gegensatz hierzu eine hohe Affinität zum Östrogenrezeptor.

Indikationen, für die die erfindungsgemäßen Verbindungen in Betracht kommen können: Hormonabhängiges Mammacarcinom, Prostatacarcinom, Endometriumscarcinom, Melanom.

Kontraindikationen: Schwangerschaft

Die pharmazeutischen Zubereitungen enthalten im allgemeinen zwischen 10 bis 500 mg, vorzugsweise 20 bis 200 mg beziehungsweise 20 bis 50 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen.

Als flüssige Anwendungsformen kommen zum Beispiel in Frage: Ölige oder alkoholische beziehungsweise wässrige Lösungen sowie Suspensionen und Emulsionen. Bevorzugte Anwendungformen sind Tabletten, die zwischen 20 und 50 mg oder Lösungen, die zwischen 0,2 bis 2 Gewichtsprozent an aktiver Substanz enthalten.

Die Einzeldosis der erfindungsgemäßen aktiven Komponenten kann beispielsweise liegen

a) bei oralen Arzneiformen zwischen 10 und 500 vorzugsweise 20 – 200 mg

b) bei parenteralen Arzneiformen (zum Beispiel intravenös, intramuskulär) zwischen 10 und 200 mg vorzugsweise 20 und 100 mg.

Beispielsweise können 3 mal täglich 1 bis 3 Tabletten mit einem Gehalt von 10 bis 50 mg wirksamer Substanz oder zum Beispiel bei intravenöser Injektion 1 bis 3 mal täglich eine Ampulle von 5 bis 20 ml Inhalt

mit 25 bis 100 mg Substanz empfohlen werden. Bei oraler Verabreichung ist die minimale tägliche Dosis beispielsweise 50 mg; die maximale tägliche Dosis bei oraler Verabreichung soll nicht über 500 mg liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei subcutaner Applikation oberhalb 20 mg/kg.

Die Herstellung eines antitumorwirksamen Arzneimittels ist dadurch gekennzeichnet, daß man Verbindungen der Formel I, worin die chemischen Symbole $R_1$ bis $R_6$, Alk, X sowie der Strukturteil A-B die in Anspruch 1 angegebene Bedeutung haben, beziehungsweise deren Salze mit physiologisch unbedenklichen Säuren zusammen mit üblichen Träger- und/oder Verdünnungs- beziehungsweise Hilfsstoffen bei Temperaturen zwischen 20 und 100°C vermischt beziehungsweise homogenisiert, die so erhaltene Mischung zur Herstellung von Zubereitungen, die in der Dosierungseinheit 10 bis 500 mg Wirkstoff der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

So werden beispielsweise die erfindungsgemäßen Verbindungen mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens einen der Hilfsstoffe Gelatine, Stärke, Polysorbat, Vinylpyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und diese Mischung zu Tabletten verpreßt, die in der Dosierungseinheit 10 bis 500 mg Wirkstoff der Formel I enthalten oder nach Zusatz von Sojalecithin bei Temperaturen zwischen 33 – 37°C in geschmolzenem Hartfett suspendiert und homogenisiert und anschließend die Mischung in Hohlzellen ausgegossen, wobei die Dosierungseinheit 10 bis 500 mg Wirkstoff enthält oder mit physiologisch unbedenklichen Säuren in Pflanzenöl bei Temperaturen zwischen 30 – 100°C aufgelöst, und die so erhaltene Lösung mit soviel Pflanzenöl aufgefüllt, daß die Endlösung 0,05 bis 2 Gewichtsprozent an Wirkstoff der Formel I enthält.

Beispiele

Allgemeine Vorschrift zur Herstellung der Platinkomplexe

Man löst 1.01 mmol des entsprechenden Diaminoindols der Formel II in 10 ml Dimethylformamid, rührt langsam und erwärmt auf 40°C. Nun werden 1.01 mmol $K_2PtCl_4$ in einem 10 ml Dimethylformamid-$H_2O$-Gemisch (5:2 Volumen/Volumen) gelöst und mit einer Pipette langsam zugetropft. Unter Lichtausschluß rührt man 24 Stunden und versetzt dann mit 2 ml Dimethylsulfoxid bis eine leichte Gelbfärbung auftritt. Nach weiteren 2 Stunden Rühren wird das Lösungsmittelgemisch im Ölpumpenvakuum abgezogen. Der braune ölige Rückstand wird mit Wasser versetzt bis blaßgelbe Kristalle ausfallen. Es wird abgesaugt und mehrmals mit Ethanol nachgewaschen. Zur Reinigung nimmt man in Dimethylformamid auf und fällt mit Ethanol/ Wasser (1:1 Volumen/Volumen) aus, saugt ab und trocknet mehrere Tage. Unter Umständen muß die Umfällung mehrmals wiederholt werden um die Komplexverbindung rein zu erhalten.

Die hergestellten Verbindungen sind in der Tabelle 1 aufgeführt. Der Indol-Teil hat für die Verbindungen der Tabelle 1 die folgende Struktur:

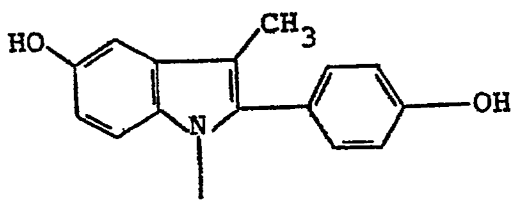

Tabelle 1

| Beispiel Nr. | Gruppe 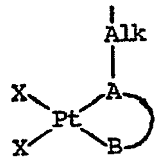 | Zersetzungs-punkt °C | Struktur Alk-A-B der Ausgangsverbindung II |
|---|---|---|---|
| 1. | 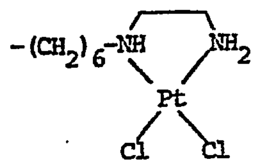 | ab 130 | $-(CH_2)_6-NH-CH_2-CH_2-NH_2$ |
| 2 | 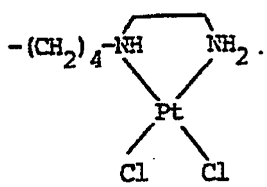 | ab 161 | $-(CH_2)_4-NH-CH_2-CH_2-NH_2$ |
| 3. | 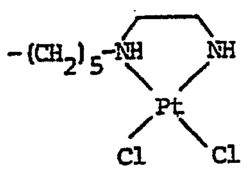 | ab 140 | $-(CH_2)_5-NH-CH_2-CH_2-NH_2$ |
| 4. | 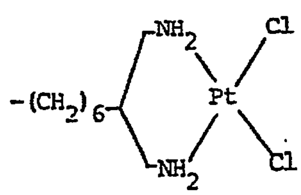 | 169 | $-(CH_2)_6-CH(CH_2NH_2)_2$ |
| 5. | 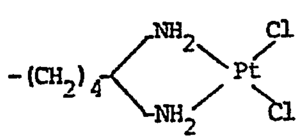 | ab 180 | $-(CH_2)_4CH(CH_2NH_2)_2$ |
| 6. | 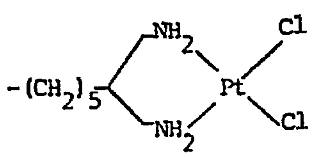 | ab 155 | $-(CH_2)_5CH(CH_2NH_2)_2$ |

| Beispiel Nr. | Gruppe 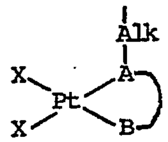 | Zersetzungspunkt °C | Struktur Alk-A-B der Ausgangsverbindung II |
|---|---|---|---|
| 7. | 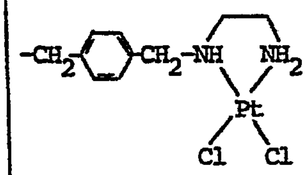 | 195–200 | 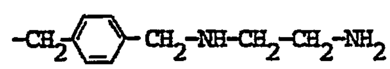 |
| 8. | 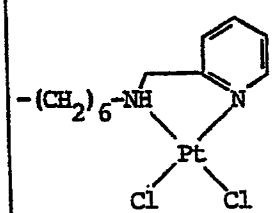 | ab 196 | 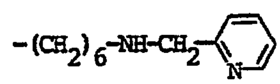 |
| 9. | 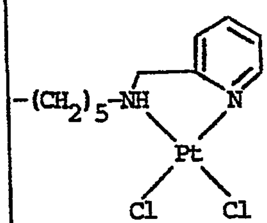 | ab 190 | 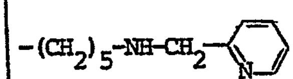 |
| 10. | 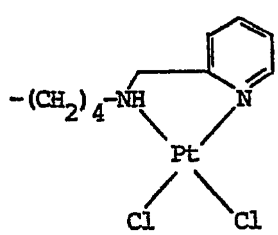 | ab 192 | 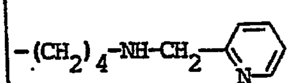 |

| Beispiel Nr. | Gruppe 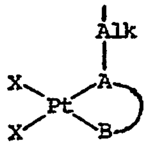 | Zersetzungspunkt °C | Struktur Alk-A-B der Ausgangsverbindung II |
|---|---|---|---|
| 11. | 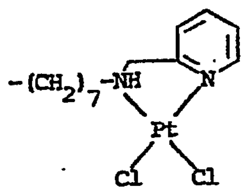 | ab 128 | 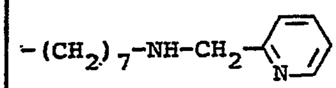 |
| 12. | 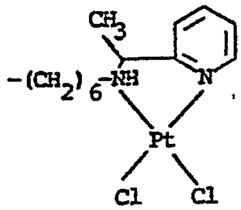 | ab 142 | 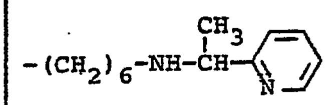 |
| 13. | 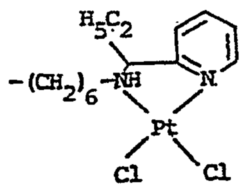 | ab 133 | 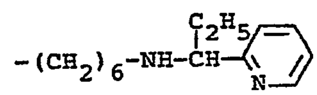 |
| 14. | 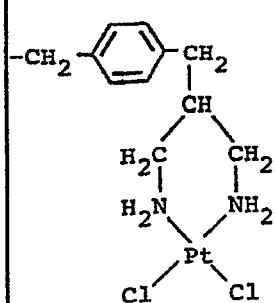 | ab 198 | 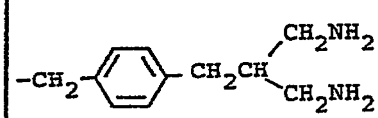 |

Herstellung der Ausgangsverbindung für Beispiel 1

1. <u>1-(6-Bromhexyl)-5-Methoxy-2-(4-methoxyphenyl)-3-methylindol</u>

In 40 ml absolutem Dimethylformamid werden 12.5 mmol (300 mg) NaH vorgelegt und auf 0°C gekühlt. Unter kräftigem Rühren werden 7.5 mmol des in 25 ml absolutem Dimethylformamid gelösten 5-Methoxy-2-(4-methoxyphenyl)-3-methyl-indols langsam zugetropft und 30 Minuten bei 0°C gerührt.

Diese Suspension wird nun unter Rühren und Eiskühlung zu einer Lösung von 11.0 mmol 1,6-Dibromhexan in 25 ml absolutem Dimethylformamid zugetropft und 30 Minuten bei 0°C gerührt. Mit Wasser wird das überschüssige NaH vernichtet, anschließend noch 2.5 Stunden bei Zimmertemperatur gerührt. Es wird mit Dichlormethan ausgeschüttelt, über $MgSO_4$ getrocknet und die Lösungsmittel abgezogen. Das

braune, ölige Rohprodukt wird an Kieselgel mit Dichlormethan chromatographiert und aus Ethanol umkristallisiert. Ausbeute 84%. F. 60°C.

2. 1-[6-(2-Amino-ethylamino)-hexyl]-5-methoxy-2-(4-methoxyphenyl)-3-methyl-indol

Unter Stickstoff-Atmosphäre werden 6.0 mmol, Ethylendiamin in 40 ml wasserfreiem Methanol gelöst. Nun werden 4.0 mmol der zuvor hergestellten 1-(6-Bromhexyl)-indolverbindung, in 80 ml absolutem Methanol, bei Raumtemperatur zugetropft. Es wird 12 Stunden unter Rückfluß erhitzt. Nach dem Abkühlen wird mit $CH_2Cl_2$ und $H_2O$ extrahiert. Nach dem Trocknen über $NA_2SO_4$ wird das Lösungsmittel abgezogen; das verbleibende farblose Öl wird anschließend im Hochvakuum destillativ gereinigt.

3. 1-(2-Amino-ethylamino)-6- [5-hxdroxy-2-(4-hydroxy- phenyl)-3-methyl-indol-1-yl]-hexan

Unter Stickstoffatmosphäre und Trockeneis/Acetonkühlung löst man 4.0 mmol des zuvor erhaltenen Dimethoxyindolderivates in 100 ml wasserfreiem Dichlormethan. Man rührt 10 Minuten und tropft dann 10.0 mmoL $BBr_3$ (gelöst in 15 ml wasserfreiem Dichlormethan) zu. Man rührt über Nacht und läßt die Reaktionsmischung auf Raumtemperatur kommen. Anschließend wird 2 Stunden unter Rückfluß erhitzt. Unter Eiskühlung und Stickstoffstrom wird nun tropfenweise mit Methanol versetzt bis die stürmische Reaktion abgeklungen ist. Nach Abziehen des Lösungsmittels wird das zurückbleibende Öl mit gesättigter $NaHCO_3$-Lösung versetzt und der anfallende Niederschlag abgesaugt. Das Rohprodukt wird in einer Soxhlethapparatur 3 – 5 Stunden mit 200 ml Triethylamin unter Rückfluß erhitzt und heiß filtriert. Nach Abziehen des Lösungsmittels wird der Rückstand in $H_2O$ suspendiert und abgesaugt (Zersetzungspunkt 112 – 114°C).

Diese Etherspaltung wird bei den Ausgangsstoffen für die anderen Beispiele analog durchgeführt.

Analog können die Ausgangsstoffe für die Beispiele 2, 3 und 7 erhalten werden.

Herstellung der Ausgangsverbindung für Beispiel 4:

1. 6-[5-Methoxy-2-(4-methoxyphenyl)-3-methyl-indol-1-yl]-hexyl-malonsäuredinitril

Unter Stickstoff-Atmosphäre und Eiskühlung werden 5.0 mmol NaH in 30 ml absolutem Tetrahydrofuran suspendiert. Sodann werden 5.0 mmol Malonsäuredinitril in 30 ml absolutem Tetrahydrofuran gelöst und langsam zugetropft. Bei Raumtemperatur wird nun 1-(6-Bromhexyl)5-methoxy-2-(4-methoxyphenyl)-3-methyl-indol, das zuvor in 30 ml absolutem Tetrahydrofuran gelöst wurde, langsam eingetropft.

Nun rührt man noch 15 Minuten bei Raumtemperatur und versetzt anschließend mit 20 ml Wasser. Man extrahiert mit Ether und Wasser, trocknet über $MgSO_4$ und destilliert die Lösungsmittel ab.

Das braune ölige Rohprodukt wird an Kieselgel mit Dichlormethan und Dichlormethan/Essigester, (10:1 Volumen/Volumen) chromatographiert. Die Umkristallisation erfolgt aus Ether.

Ausbeute: 48%, F. 93 – 95°C.

2. 1-Amino-2-aminomethyl-6-[5-methoxy-2-(4-methoxyphenyl)-3-methyl-indol-1-yl]-octan

In 40 ml absolutem Tetrahydrofuran werden 9.5 mmol $LiAlH_4$ unter Eiskühlung suspendiert. Dazu werden 2.5 mmol des in 30 ml absolutem Tetrahydrofuran gelösten Dinitrils unter kräftigem Rühren langsam zugetropft. Man kocht das Reaktionsgemisch 30 Stunden bei 100°C unter Rückfluß. Nach dem Abkühlen wird unter Eiskühlung mit 60 ml Ether versetzt, und zur Vernichtung von überschüssigem $LiAlH_4$ mit Wasser versetzt.

Man saugt vom Niederschlag ab, trocknet das Filtrat über KOH und $K_2CO_3$ und rotiert die Lösungsmittel ab. Das so erhaltene Produkt ist ein farbloses bis schwach gelbgefärbtes, zähflüssiges Öl. Die Umsetzung kann IR-spektroskopisch am Verschwinden der CN-Bande bei 2265 $cm^{-1}$ und dem Erscheinen der $NH_2$-Bande bei 3380 $cm^{-1}$ verfolgt werden.

Ausbeute: 80%.

Durch Abspaltung der beiden Methoxygruppen (analog wie für Beispiel 1 angegeben) erhält man die Ausgangsverbindung II für das Beispiel 4:
1-Amino-2-aminomethyl-6-[5-hydroxy-2-(4-hydroxyphenyl)-3-methyl-indol-1-yl]-octan.

Die Ausgangsstoffe für die Beispiele 5 und 6 können analog hergestellt werden.

Herstellung der Ausgangsverbindung für Beispiel 8:

1. 1-(6-Amino-hexyl)-5-methoxy-2-(4-methoxyphenyl)-3-methyl-indol

8.0 mmol 1-(6-Bromhexyl)-5-methoxy-2-(4-methoxyphenyl)-3-methyl-indol und 8.8 mmol Phthalamid-Kalium werden in 100 ml wasserfreiem Dimethylformamid 2 Stunden unter Rückfluß erhitzt. Nach dem Abkühlen wird mit Dichlormethan und $H_2O$ ausgeschüttelt. Anschließend wird über $Na_2SO_4$ getrocknet und das Lösungsmittel abdestilliert. Der Rückstand wird an Kieselgel mit Dichlormethan chromatographiert.

Zur Freisetzung des Amins werden die erhaltenen farblosen Kristalle in 50 ml Ethanol (99%) aufgenommen und mit Hydrazin-Hydrat in 20 ml Ethanol versetzt. Nun wird 2 Stunden unter Rückfluß erwärmt; nach dem Abkühlen wird mit 40 ml 2 N HCl-Lösung angesäuert und vom Niederschlag abgesaugt. Nach dem Abdestillieren des Lösungsmittels wird mit 40 ml 2 N NaOH alkalisch gemacht und dreimal mit Essigester extrahiert. Anschließend wird über $Na_2SO_4$ getrocknet und an Kieselgel mit Dichlormethan/Essigester (10:1, Volumen/Volumen) chromatographiert. Man erhält ein farbloses bis gelbgefärbtes zähflüssiges Öl.

2. 5-Methoxy-2-(4-methoxyphenyl)-3-methyl-1-[6-(2-pyridyl-methylamino)-hexyl]-indol

Darstellung der Schiff'schen Base:
10.0 mmol 2-Pyridinaldehyd werden mit 11.0 mmol des nach 1. erhaltenen 6-Aminohexyl-indols in 60 ml trockenem Benzol vorgelegt und in einer Soxhletapparatur mit wasserfreiem $CaSO_4$ 24 Stunden unter Rückfluß erhitzt. Nach Abziehen des Lösungsmittels wird das als zähes Öl anfallende Kondensationsprodukt in Diethylether aufgenommen und über $Na_2SO_4$ getrocknet. Durch Kugelrohrdestillation im Hochvakuum erhält man die reine Schiff'sche Base als gelbes zähflüssiges Öl.

Reduktion der Schiff'schen Base zum Amin:
Eine auf −10°C gekühlte Lösung von 10.0 mmol der Schiff'schen Base in 40 ml wasserfreiem Methanol wird mit 12.0 mmol $NaBH_4$ versetzt. Innerhalb von 2 Stunden läßt man auf Raumtemperatur erwärmen und rührt anschließend 12 Stunden bei 50°C. Nach Abziehen des Methanols gibt man 20 ml $H_2O$ zu, schüttelt dreimal mit Ether aus und trocknet über $Na_2SO_4$. Nach Kugelrohrdestillation im Hochvakuum erhält man das Amin als gelbes zähflüssiges Öl.

Abspaltung der Methoxygruppen erfolgt in gleicher Weise wie bei Beispiel 1.

Die Ausgangsstoffe der Formel II für die übrigen Beispiele der Tabelle 1 können analog erhalten werden.

Tabelle 2 betrifft Verbindungen mit verschiedenem Indolteil. Die Alk-diamino-dichlorplatingruppe hat bei den Beispielen 15 – 17 dieselbe Struktur wie bei Beispiel 1:

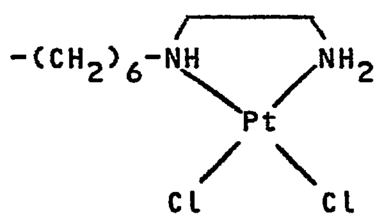

Bei den Beispielen 18 und 19 hat die Alk-diamino-chloroplatingruppe dieselbe Struktur wie bei Beispiel 2:

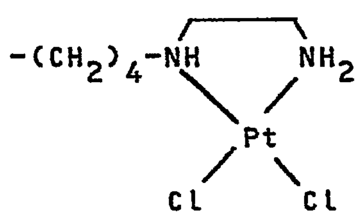

Tabelle 2

| Beispiel Nr. | Indolteil | Zersetzungs-punkt °C | Struktur Alk-A-B der Ausgangsverbindung II |
|---|---|---|---|
| 15. | 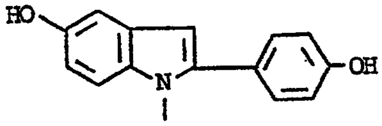 | ab 155 | wie Beispiel 1 |
| 16. | 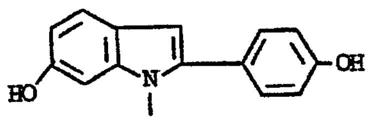 | ab 160 | wie Beispiel 1 |
| 17. | 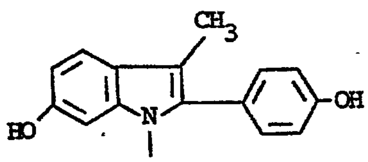 | ab 128 | wie Beispiel 1 |
| 18. | 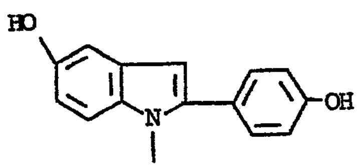 | ab 140 | wie Beispiel 2 |
| 19. | 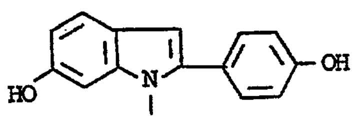 | ab 104 | wie Beispiel 2 |

Beispiele für pharmazeutische Zubereitungen

Beispiel für Tabletten

200 g der Verbindung gemäß Beispiel 2, 500 g Lactose, 360 g mikronisierte Cellulose, 130 g Maisstärke und 10 g Magnesiumstearat werden durch ein Sieb mit einer Maschenweite von 1,0 mm gegeben und in einem geeigneten Mischer homogenisiert. Diese Masse wird in bekannter Weise zu Tabletten von 120 mg verpreßt.

1 Tablette enthält 20 mg Wirkstoff.

Beispiel für überzogene Tabletten

Zur Herstellung überzogener Tabletten werden Tabletten entsprechend Beispiel 1 in bekannter Weise mit Hilfe einer Sprüheinrichtung mit einem magen- oder dünndarmlöslichen Film überzogen, der aus einem geeigneten polymeren Filmbildner wie zum Beispiel Estern von Acrylaten oder Methacrylaten und geeigneten Hilfsstoffen wie Netzmittel, Weichmachern, Farbstoffen, Gleitmitteln usw. bestehen kann. Die Tabletten können auch in bekannter Weise zu Dragees verarbeitet werden.

Beispiel für Trockensubstanz zur Injektion

2 kg der Verbindung gemäß Beispiel 1 werden unter sterilen Bedingungen auskristallisiert, getrocknet und in einer geeigneten Mühle (zum Beispiel Condux Mühle) steril gemahlen. Die Korngröße der abfüllfähigen Substanz sollte zwischen 50 µm und 250 µm liegen. Der sterile, gemahlene Wirkstoff wird mit einem geeigneten Schneckendosiergerät zu 100 mg in 40 ml Injektionsflaschen abgefüllt. Zur intravenösen Anwendung wird die Trockensubstanz in 10 ml eines geeigneten Lösungsmittels (zum Beispiel Polyethylenglycol 400/0,9%ige wäßrige Kochsalzlösung 1:1) gelöst. Zu jeder Injektionsflasche wird eine entsprechende Ampulle mit Lösungsmitteln bereitgestellt.
Eine 10 ml Injektionsflasche enthält 100 mg Wirkstoff.
Eine Ampulle enthält 10 ml Lösungsmittel.

**Patentansprüche für die Vertragsstaaten: BE, CH, DE, FR, GB, IT, LI, LU, NL, SE**

1. cis-Platin-Komplexe der allgemeinen Formel

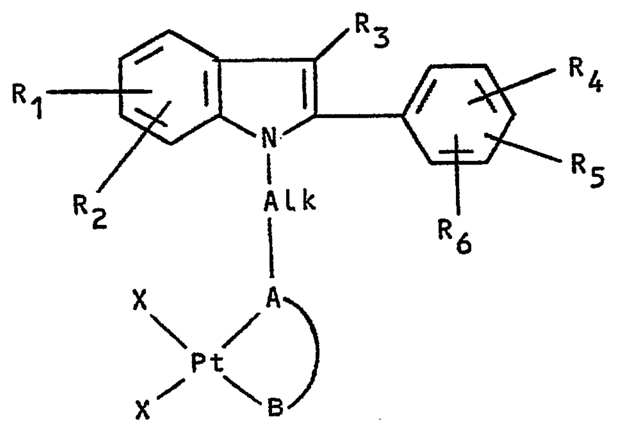

I

worin $R_1$ Wasserstoff, eine Hydroxygruppe oder eine $C_2$–$C_6$-Alkanoyloxygruppe, $R_2$ Wasserstoff oder ein Halogenatom, $R_3$ Wasserstoff oder eine $C_1$–$C_6$-Alkylgruppe, $R_4$ eine Hydroxygruppe oder eine $C_2$–$C_6$-Alkanoyloxygruppe, $R_5$ Wasserstoff oder ein Halogenatom und $R_6$ Wasserstoff oder ein Halogenatom bedeutet, Alk eine $C_2$–$C_{10}$-Alkylenkette darstellt, wobei 4 benachbarte $CH_2$-Gruppen auch durch einen 1,4-Phenylenring ersetzt sein können, A–B die Gruppe $HN$-$CH_2$-$CH_2$-$NH_2$, $H_2N$-$CH_2$-$CH$-$CH_2$-$NH_2$ oder

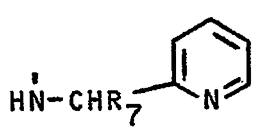

bedeutet, und $R_7$ ein Wasserstoffatom, eine $C_1$–$C_6$-Alkylgruppe oder ein Phenylrest ist und X für das Äquivalent eines physiologisch verträglichen Anions steht.
2. Verfahren zur Herstellung von cis-Platin-Komplexen der allgemeinen Formel

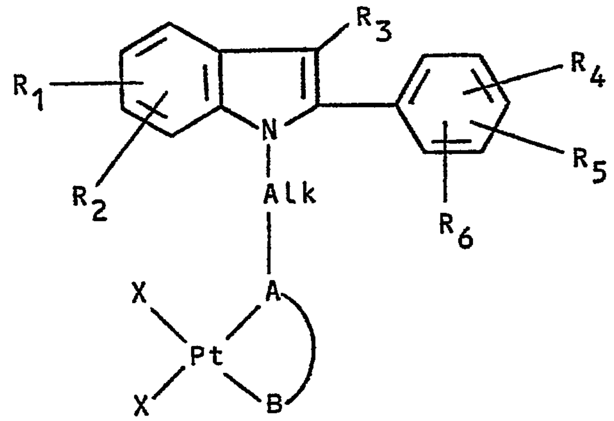

I

worin $R_1$ Wasserstoff, eine Hydroxygruppe oder eine $C_2$–$C_6$-Alkanoyloxygruppe, $R_2$ Wasserstoff oder ein Halogenatom, $R_3$ Wasserstoff oder eine $C_1$–$C_6$-Alkylgruppe, $R_4$ eine Hydroxygruppe oder eine $C_2$–$C_6$-Alkanoyloxygruppe, $R_5$ Wasserstoff oder ein Halogenatom und $R_6$ Wasserstoff oder ein Halogenatom bedeutet, Alk eine $C_2$–$C_{10}$-Alkylenkette darstellt, wobei 4 benachbarte $CH_2$-Gruppen auch durch einen 1,4-Phenylenring ersetzt sein können, A–B die Gruppe $HN\text{-}CH_2\text{-}CH_2\text{-}NH_2$, $H_2N\text{-}CH_2\text{-}CH\text{-}CH_2\text{-}NH_2$ oder

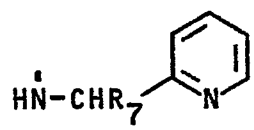

bedeutet, und $R_7$ ein Wasserstoffatom, eine $C_1$–$C_6$-Alkylgruppe oder ein Phenylrest ist und X für das Äquivalent eines physiologisch verträglichen Anions steht, dadurch gekennzeichnet, daß man eine Tetrahalogeno-platin(II)-säure, ein Tetrahalogeno-platin(II)-Komplexsalz mit zwei einwertigen oder einem zweiwertigen Kation oder ein Platin(II)-halogenid mit einer Verbindung der Formel

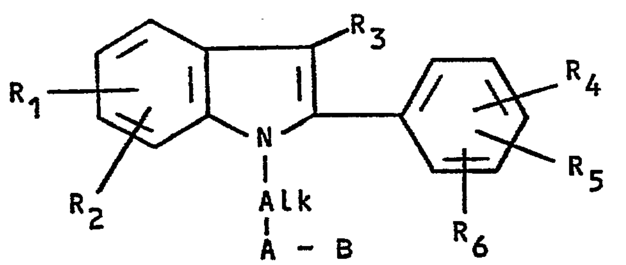

II

oder einem Salz der Verbindung II umsetzt, wobei die Reste $R_1$, $R_2$, $R_3$, $R_4$, $R_5$, $R_6$ und die Gruppen Alk und A - B die angegebenen Bedeutungen haben und gegebenenfalls in einer erhaltenen Verbindung der Formel I den Rest X beziehungsweise die Reste X gegen andere physiologisch verträgliche Anionen austauscht.

3. Arzneimittel, enthaltend eine Verbindung der allgemeinen Formel I neben üblichen Träger- und/oder Verdünnungs- beziehungsweise Hilfsstoffen.

4. Verfahren zur Herstellung eines Arzneimittels, dadurch gekennzeichnet, daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

5. Verwendung von Verbindungen der allgemeinen Formel I zur Herstellung von Arzneimitteln.

**Patentansprüche für die Vertragsstaaten AT, ES, GR**

1. Verfahren zur Herstellung von cis-Platin-Komplexen der allgemeinen Formel

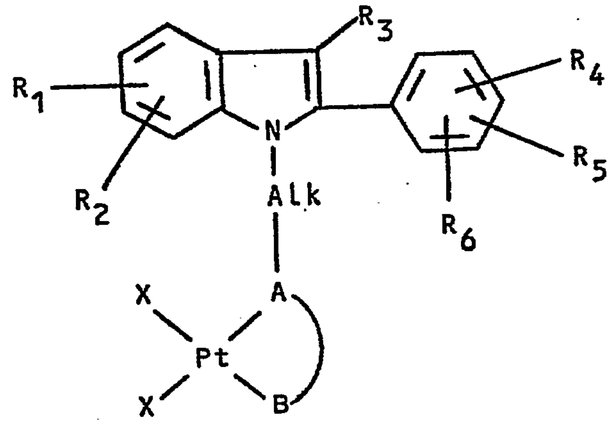

I

worin $R_1$ Wasserstoff, eine Hydroxygruppe oder eine $C_2$–$C_6$-Alkanoyloxygruppe, $R_2$ Wasserstoff oder, ein Halogenatom, $R_3$ Wasserstoff oder eine $C_1$–$C_6$-Alkylgruppe, $R_4$ eine Hydroxygruppe oder eine $C_2$–$C_6$-Alkanoyloxygruppe, $R_5$ Wasserstoff oder ein Halogenatom und $R_6$ Wasserstoff oder ein Halogenatom bedeutet, Alk eine $C_2$–$C_{10}$-Alkylenkette darstellt, wobei 4 benachbarte $CH_2$-Gruppen auch durch einen 1,4-Phenylenring ersetzt sein können, A–B die Gruppe HN-$CH_2$-$CH_2$-$NH_2$, $H_2N$-$CH_2$-CH-$CH_2$-$NH_2$ oder

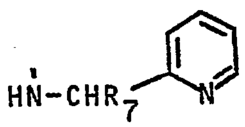

bedeutet, und $R_7$ ein Wasserstoffatom, eine $C_1$–$C_6$-Alkylgruppe oder ein Phenylrest ist und X für das Äquivalent eines physiologisch verträglichen Anions steht, dadurch gekennzeichnet, daß man eine Tetrahalogeno-platin(II)-säure, ein Tetrahalogeno-platin(II)-Komplexsalz mit zwei einwertigen oder einem zweiwertigen Kation oder ein Platin(II)-halogenid mit einer Verbindung der Formel

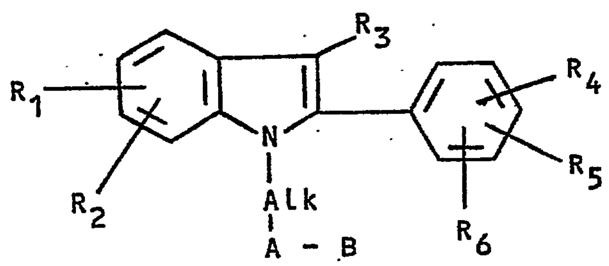

II

oder einem Salz der Verbindung II umsetzt, wobei die Reste $R_1$, $R_2$, $R_3$, $R_4$, $R_5$, $R_6$ und die Gruppen Alk und A - B die angegebenen Bedeutungen haben und gegebenenfalls in einer erhaltenen Verbindung der Formel I den Rest X beziehungsweise die Reste X gegen andere physiologisch verträgliche Anionen austauscht.

2. Verfahren zur Herstellung eines Arzneimittels, enthaltend als Wirkstoff mindestens eine Verbindung der allgemeinen Formel I, worin die chemischen Symbole $R_1$ bis $R_6$, Alk, X sowie der Strukturteil A-B die in Anspruch 1 angegebene Bedeutung haben, dadurch gekennzeichnet, daß mindestens eine Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

3. Verfahren zur Herstellung eines antitumorwirksamen Arzneimittels, dadurch gekennzeichnet, daß man Verbindungen der Formel I, worin die chemischen Symbole $R_1$ bis $R_6$, Alk, X sowie der Strukturteil A–B die in Anspruch 1 angegebene Bedeutung haben, beziehungsweise deren Salze mit physiologisch unbedenklichen Säuren zusammen mit üblichen Träger- und/oder Verdünnungs- beziehungsweise Hilfsstoffen bei Temperaturen zwischen 20 und 100°C vermischt beziehungsweise homogenisiert, die so erhaltene Mischung zur Herstellung von Zubereitungen, die in der Dosierungseinheit 10 bis 500 mg Wirkstoff der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

4. Verfahren zur Herstellung eines antitumorwirksamen Arzneimittels, dadurch gekennzeichnet, daß man Verbindungen der Formel I, worin die chemischen Symbole $R_1$ bis $R_6$, Alk, X sowie der Strukturteil A-B die in Anspruch 1 angegebene Bedeutung haben, beziehungsweise deren Salze mit physiologisch unbedenklichen Säuren mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum vermischt, die erhaltene Mischung, gegebenenfalls mit einer wässrigen Lösung, die als Bestandteil mindestens einen der Hilfsstoffe Gelatine, Stärke, Polysorbat, Vinylpyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und diese Mischung zu Tabletten verpreßt, die in der Dosierungseinheit 10 bis 500 mg Wirkstoff der Formel I enthalten.

5. Verfahren zur Herstellung eines antitumorwirksamen Arzneimittels, dadurch gekennzeichnet, daß man Verbindungen der Formel I oder deren Salze mit physiologisch unbedenklichen Säuren nach Zusatz von Sojalecithin bei Temperaturen zwischen 33 – 37°C in geschmolzenem Hartfett suspendiert und homogenisiert und anschließend die Mischung in Hohlzellen ausgießt, wobei die Dosierungseinheit 10 bis 500 mg Wirkstoff enthält.

6. Verfahren zur Herstellung einer antitumorwirksamen Injektionslösung, dadurch gekennzeichnet, daß man Verbindungen der Formel I oder deren Salze mit physiologisch unbedenklichen Säuren in Pflanzenöl bei Temperaturen zwischen 30–100°C auflöst, und die so erhaltene Lösung mit soviel Pflanzenöl auffüllt, daß die Endlösung 0,05 bis 2 Gewichtsprozent an Wirkstoff der Formel I enthält.

7. Verwendung von Verbindungen der allgemeinen Formel I, worin $R_1$ Wasserstoff, eine Hydroxygruppe oder eine $C_2$–$C_6$-Alkanoyloxygruppe, $R_2$ Wasserstoff oder ein Halogenatom, $R_3$ Wasserstoff oder eine $C_1$–$C_6$-Alkylgruppe, $R_4$ eine Hydroxygruppe oder eine $C_2$–$C_6$-Alkanoyloxygruppe, $R_5$ Wasserstoff oder ein Halogenatom und $R_6$ Wasserstoff oder ein Halogenatom bedeutet, Alk eine $C_2$–$C_{10}$-Alkylenkette darstellt, wobei 4 benachbarte $CH_2$-Gruppen auch durch einen 1,4-Phenylenring ersetzt sein können, A–B die Gruppe HN-$CH_2$-$CH_2$-$NH_2$, $H_2N$-$CH_2$-CH-$CH_2$-$NH_2$ oder

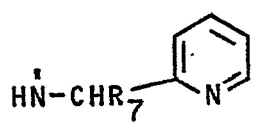

bedeutet, und $R_7$ ein Wasserstoffatom, eine $C_1$–$C_6$-Alkylgruppe oder ein Phenylrest ist und X für das Äquivalent eines physiologisch verträglichen Anions steht, zur Herstellung von Arzneimitteln.

**Claims for the contracting States BE, CH, DE, FR, GB, IT, LI, LU, NL, SE**

1. Cis-platinum complexes of the general formula

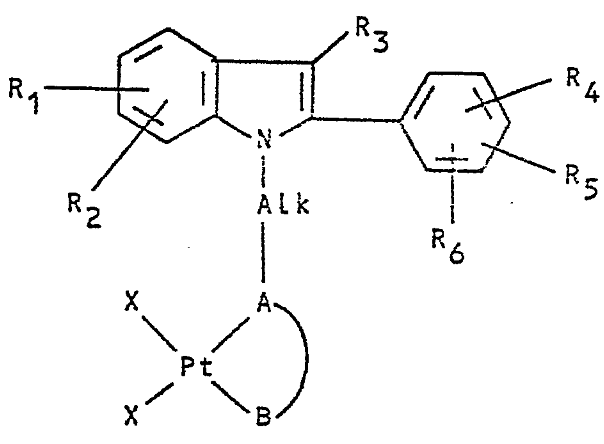

I

wherein $R_1$ denotes hydrogen, a hydroxyl group or a $C_2$–$C_6$- alkanoyloxy group, $R_2$ denotes hydrogen or a halogen atom, $R_3$ denotes hydrogen or a $C_1$–$C_6$-alkyl group, $R_4$ denotes a hydroxyl group or a $C_2$–$C_6$-alkanoyloxy group, $R_5$ denotes hydrogen or a halogen atom and $R_6$ denotes hydrogen or a halogen atom, Alk represents a $C_2$–$C_{10}$-alkylene chain, where 4 adjacent $CH_2$ groups may furthermore be replaced by a 1,4-phenylene ring, A–B denotes the group HN-$CH_2$-$CH_2$-$NH_2$, $H_2N$-$CH_2$-CH-$CH_2$-$NH_2$ or

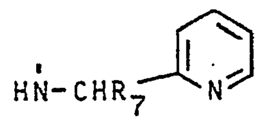

and $R_7$ is a hydrogen atom, a $C_1$–$C_6$-alkyl group or a phenyl radical and X represents one equivalent of a physiologically tolerated anion.

2. Process for the preparation of cis-platinum complexes of the general formula

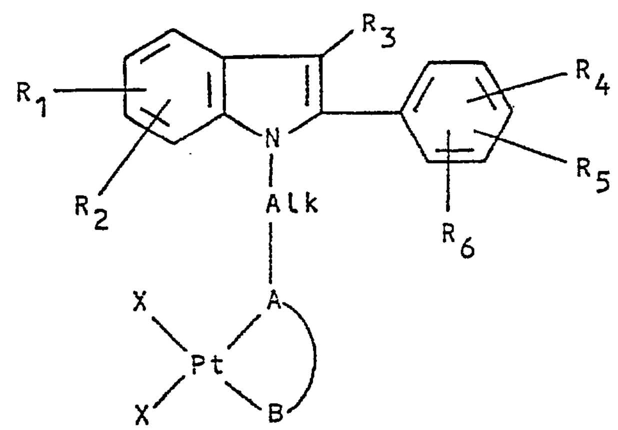

I

wherein $R_1$ denotes hydrogen, a hydroxyl group or a $C_2$–$C_6$-alkanoyloxy group, $R_2$ denotes hydrogen or a halogen atom, $R_3$ denotes hydrogen or a $C_1$–$C_6$-alkyl group, $R_4$ denotes a hydroxyl group or a $C_2$–$C_6$-alkanoyloxy group, $R_5$ denotes hydrogen or a halogen atom and $R_6$ denotes hydrogen or a halogen atom, Alk represents a $C_2$–$C_{10}$-alkylene chain, where 4 adjacent $CH_2$ groups may furthermore be replaced by a 1,4-phenylene ring, A–B denotes the group HN-$CH_2$-$CH_2$-$NH_2$, $H_2N$-$CH_2$-CH-$CH_2$-$NH_2$ or

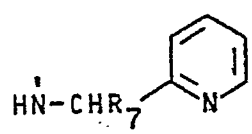

and $R_7$ is a hydrogen atom, a $C_1$–$C_6$-alkyl group or a phenyl radical and X represents one equivalent of a physiologically tolerated anion, characterised in that a tetrahaloplatinum(II) acid, a tetrahaloplatinum(II) complex salt having two monovalent cations or one divalent cation or a platinum(II) halide is reacted with a compound of the formula

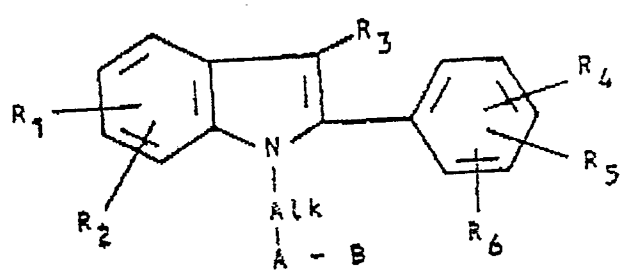

II

or a salt of the compound II, wherein the radicals $R_1$, $R_2$, $R_3$, $R_4$, $R_5$ and $R_6$ and the groups Alk and A–B have the stated meanings, and, if required, the radical X or the radicals X in a resulting compound of the formula I is or are exchanged for other physiologically tolerated anions.

3. Medicament containing a compound of the general formula I in addition to customary carriers and/or diluents or excipients.

4. Process for the preparation of a medicament, characterised in that a compound of the general formula I is processed with conventional pharmaceutical carriers or diluents or other excipients to give pharmaceutical formulations, or is brought into a therapeutically usable form.

5. Use of compounds of the general formula I for the preparation of medicaments.

**Claims for the contracting States AT, ES, GR**

1. Process for the preparation of cis-platinum complexes of the general formula

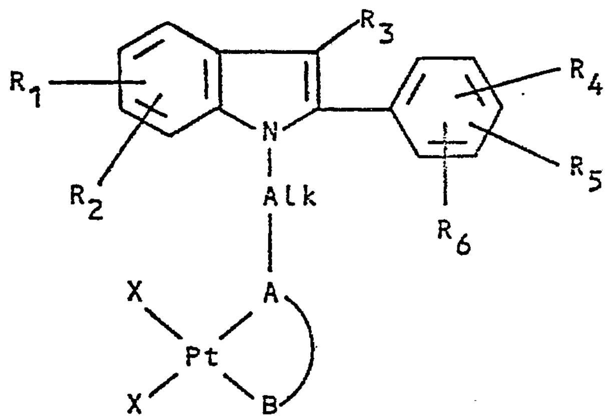

I

wherein $R_1$ denotes hydrogen, a hydroxyl group or a $C_2$–$C_6$-alkanoyloxy group, $R_2$ denotes hydrogen or a halogen atom, $R_3$ denotes hydrogen or a $C_1$–$C_6$-alkyl group, $R_4$ denotes a hydroxyl group or a $C_2$–$C_6$-alkanoyloxy group, $R_5$ denotes hydrogen or a halogen atom and $R_6$ denotes hydrogen or a halogen atom, Alk represents a $C_2$–$C_{10}$-alkylene chain, where 4 adjacent $CH_2$ groups may furthermore be replaced by a 1,4-phenylene ring, A–B denotes the group $HN\text{-}CH_2\text{-}CH_2\text{-}NH_2$, $H_2N\text{-}CH_2\text{-}CH\text{-}CH_2\text{-}NH_2$ or

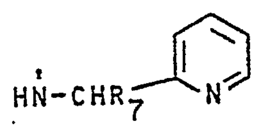

and $R_7$ is a hydrogen atom, a $C_1$–$C_6$-alkyl group or a phenyl radical and X represents one equivalent of a physiologically tolerated anion, characterised in that a tetrahaloplatinum(II) acid, a tetrahaloplatinum(II) complex salt having two monovalent cations or one divalent cation or a platinum(II) halide is reacted with a compound of the formula

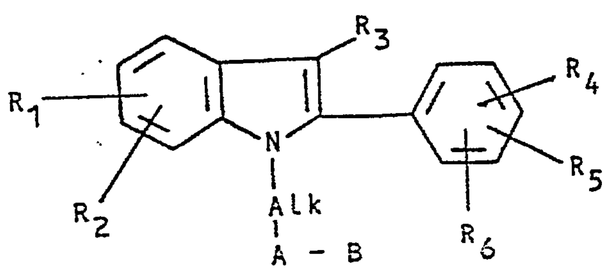

II

or a salt of the compound II, wherein the radicals $R_1$, $R_2$, $R_3$, $R_4$, $R_5$ and $R_6$ and the groups Alk and A-B have the stated meanings, and, if required, the radical X or the radicals X in a resulting compound of the formula I is or are exchanged for other physiologically tolerated anions.

2. Process for the preparation of a medicament, containing at least one compound of the general formula I, wherein the chemical symbols $R_1$ to $R_6$, Alk, X and the structural moiety A–B have the meaning stated in claim 1, characterised in that at least one compound of the formula I is processed with conventional pharmaceutical carriers and/or diluents or other excipients to give pharmaceutical formulations, or is brought into a therapeutically usable form.

3. Process for the preparation of a medicament having antitumor activity, characterised in that compounds of the formula I, wherein the chemical symbols $R_1$ to $R_6$, Alk, X and the structural moiety A–B have the meaning stated in claim 1, or their salts with physiologically acceptable acids are mixed or homogenised together with customary carriers and/or diluents or excipients at temperatures between 20 and 100°C, and, for the preparation of formulations which contain 10 to 500 mg of active ingredient of the formula I in the unit dose, the mixture thus obtained is poured into hollow cells of appropriate size or filled into capsules of appropriate size or granulated and then pressed to give tablets, optionally with the addition of further customary excipients.

4. Process for the preparation of a medicament having antitumor activity, characterised in that compounds of the formula I, wherein the chemical symbols $R_1$ to $R_6$, Alk, X and the structural moiety A–B have the meaning stated in claim 1, or their salts with physiologically acceptable acids are mixed with one or more of the following substances: starch, cellulose, lactose, formalin-casein, modified starch, magnesium stearate, calcium hydrogen phosphate, finely divided silica or talc, the mixture obtained is granulated, optionally with an aqueous solution which contains at least one of the excipients gelatine, starch, polysorbate, vinylpyrrolidone/vinyl acetate copolymer and/or polyoxyethylene sorbitan monooleate as a constituent, the granules are optionally homogenised with one or more of the above-mentioned excipients and this mixture is pressed to give tablets which contain 10 to 500 mg of active ingredient of the formula I in the unit dose.

5. Process for the preparation of a medicament having antitumor activity, characterised in that the compounds of the formula I or their salts with physiologically active acids are suspended in molten hard fat at temperatures between 33 and 37°C, after the addition of soya lecithin, and homogenised, and the mixture is then poured into hollow cells, the unit dose containing 10 to 500 mg of active ingredient.

6. Process for the preparation of an injection solution having antitumor activity, characterised in that compounds of the formula I or their salts with physiologically acceptable acids are dissolved in vegetable oil at temperatures between 30 and 100°C, and the solution thus obtained is made up with an amount of vegetable oil such that the final solution contains 0.05 to 2 percent by weight of active ingredient of the formula I.

7. Use of compounds of the general formula I, wherein $R_1$ denotes hydrogen, a hydroxyl group or a $C_2$–$C_6$-alkanoyloxy group, $R_2$ denotes hydrogen or a halogen atom, $R_3$ denotes hydrogen or a $C_1$–$C_6$-alkyl group, $R_4$ denotes a hydroxyl group or a $C_2$–$C_6$-alkanoyloxy group, $R_5$ denotes hydrogen or a halogen atom and $R_6$ denotes hydrogen or a halogen atom, Alk represents a $C_2$–$C_{10}$-alkylene chain, where 4 adjacent $CH_2$ groups may furthermore be replaced by a 1,4-phenylene ring, A–B denotes the group HN-$CH_2$-$CH_2$-$NH_2$, $H_2N$-$CH_2$-CH-$CH_2$-$NH_2$ or

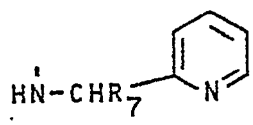

and $R_7$ is a hydrogen atom, a $C_1$–$C_6$-alkyl group or a phenyl radical and X represents one equivalent of a physiologically tolerated anion, for the preparation of medicaments.

**Revendications pour les Etats contractants BE, CH, DE, FR, GB, IT, LI, LU, NL, SE**

1. Cis-complexes de platine de formule générale

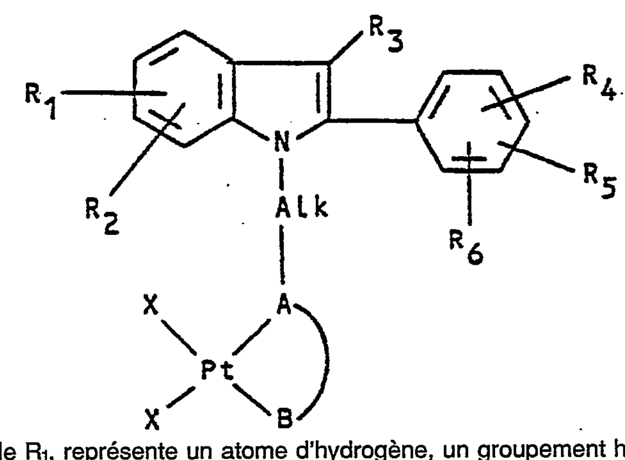

I

dans laquelle $R_1$, représente un atome d'hydrogène, un groupement hydroxy ou un groupement alcanoyloxy en $C_2$–$C_6$, $R_2$ représente un atome d'hydrogène ou un atome d'halogène, $R_3$ représente un atome d'hydrogène ou un groupement alkyle en $C_1$–$C_6$, $R_4$ représente un groupement hydroxy ou un groupement alcanoyloxy en $C_2$–$C_6$, $R_5$ représente un atome d'hydrogène ou un atome d'halogène et $R_6$ représente un atome d'hydrogène ou un atome d'halogène, Alk représente une chaîne alkylène en $C_2$–$C_{10}$, 4 groupements $CH_2$ voisins pouvant aussi être remplacés par un noyau 1,4-phénylène, A–B représente le groupement HN–$CH_2$–$CH_2$–$NH_2$, $H_2N$–$CH_2$–CH–$CH_2$–$NH_2$ ou

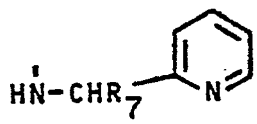

$R_7$ étant un atome d'hydrogène, un groupement alkyle en $C_1$–$C_6$ ou un reste phényle, et X est mis pour un équivalent d'un anion acceptable physiologiquement.

2. Procédé de préparation de cis-complexes de platine de formule générale

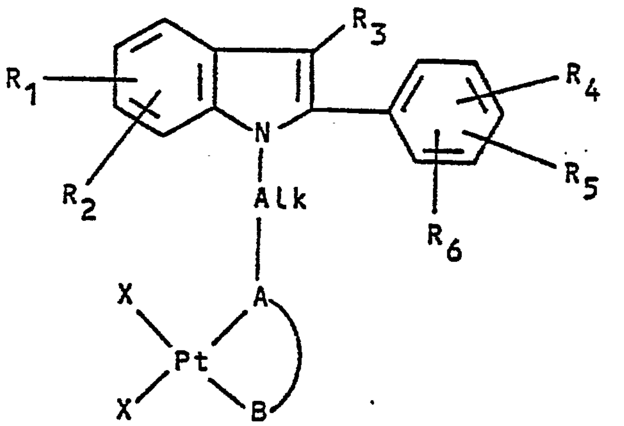

I

dans laquelle $R_1$, représente un atome d'hydrogène, un groupement hydroxy ou un groupement alcanoyloxy en $C_2$–$C_6$, $R_2$ représente un atome hydrogène ou un atome d'halogène, $R_3$ représente un atome d'hydrogène ou un groupement alkyle en $C_1$–$C_6$, $R_4$ représente un groupement hydroxy ou un groupement alcanoyloxy en $C_2$–$C_6$, $R_5$ représente un atome d'hydrogène ou un atome d'halogène et $R_6$ représente un atome d'hydrogène ou un atome d'halogène, Alk représente une chaîne alkylène en $C_2$–$C_{10}$, 4 groupements $CH_2$ voisins pouvant aussi être remplacés par un noyau 1,4-phénylène, A–B représente le groupement HN–$CH_2$–$CH_2$–$NH_2$, $H_2N$–$CH_2$–CH–$CH_2$–$NH_2$ ou

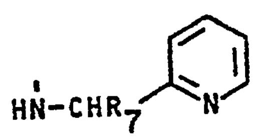

$R_7$ étant un atome d'hydrogène, un groupement alkyle en $C_1$–$C_6$ ou un reste phényle, et X est mis pour un équivalent d'un anion acceptable physiologiquement, caractérisé en ce qu'on fait réagir un acide tétrahalogéno-platineux, un sel complexe de tétrahalogéno-platine(II) avec deux cations monovalents ou un cation bivalent ou un halogénure de platine(II) avec un composé de formule

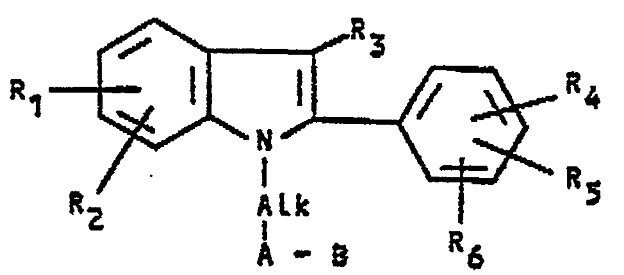

II

ou un sel du composé II, les restes $R_1$, $R_2$, $R_3$, $R_4$, $R_5$, $R_6$ et les groupements Alk et A – B ayant les significations indiquées et éventuellement, dans un composé de formule I obtenu, on remplace le reste X ou les restes X par d'autres anions acceptables physiologiquement.

3. Médicament contenant un composé de formule générale I, outre des excipients et/ou diluants ou adjuvants usuels.

4. Procédé de préparation d'un médicament, caractérisé en ce qu'un composé de formule générale I est transformé en préparations pharmaceutiques ou mis sous une forme applicable à des fins thérapeutiques, avec des excipients, diluants ou autres adjuvants pharmaceutiques.

5. Utilisation de composés de formule générale I pour la préparation de médicaments.

**Revendications pour les Etats contractants AT, ES, GR**

1. Procédé de préparation de cis-complexes de platine de formule générale

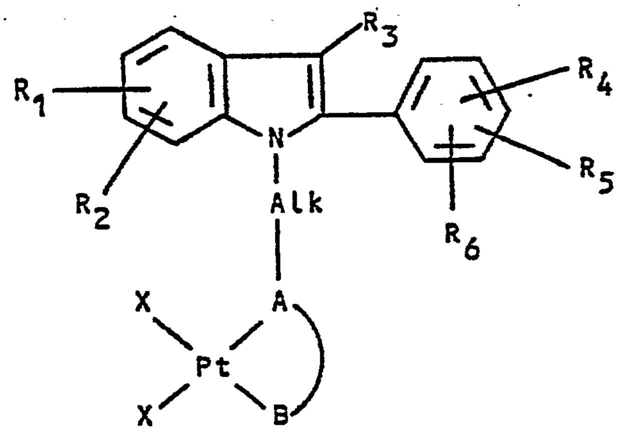

I

dans laquelle $R_1$ représente un atome d'hydrogène, un groupement hydroxy ou un groupement alcanoyloxy en $C_2$–$C_6$, $R_2$ représente un atome d'hydrogène ou un atome d'halogène, $R_3$ représente un atome d'hydrogène ou un groupement alkyle en $C_1$–$C_6$, $R_4$ représente un groupement hydroxy ou un groupement alcanoyloxy en $C_2$–$C_6$, $R_5$ représente un atome d'hydrogène ou un atome d'halogène et $R_6$ représente un atome d'hydrogène ou un atome d'halogène, Alk représente une chaîne alkylène en $C_2$–$C_{10}$, 4 groupements $CH_2$ voisins pouvant aussi être remplacés par un noyau 1,4-phénylène, A–B représente le groupement HN–$CH_2$–$CH_2$–$NH_2$, $H_2N$–$CH_2$–CH–$CH_2$–$NH_2$ ou

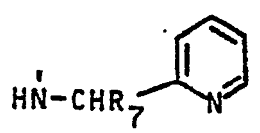

$R_7$ étant un atome d'hydrogène, un groupement alkyle en $C_1$–$C_6$ ou un reste phényle, et X est mis pour un équivalent d'un anion acceptable physiologiquement, caractérisé en ce qu'on fait réagir un acide tétrahalogéno-platineux, un sel complexe de tétrahalogéno-platine(II) avec deux cations monovalents ou un cation bivalent ou un halogénure de platine(II) avec un composé de formule

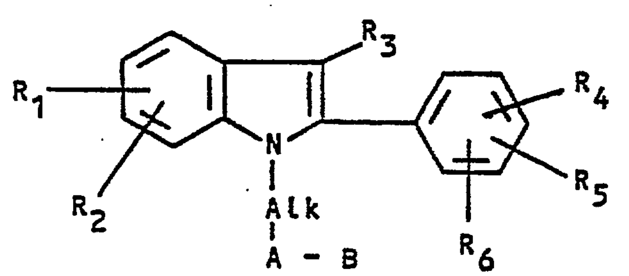

II

ou un sel du composé II, les restes $R_1$, $R_2$, $R_3$, $R_4$, $R_5$, $R_6$ et les groupements Alk et A – B ayant les significations indiquées et éventuellement, dans un composé de formule I obtenu, on remplace le reste X ou les restes X par d'autres anions acceptables physiologiquement.

2. Procédé de préparation d'un médicament contenant, en tant que substance active, au moins un composé de formule I dans laquelle les symboles chimiques $R_1$ à $R_6$, Alk, X, ainsi que la partie structurale A–B ont les significations indiquées dans la revendication 1, caractérisé en ce qu'au moins un composé de formule générale I est transformé en préparations pharmaceutiques ou mis sous une forme applicable à des fins thérapeutiques, avec des excipients, diluants ou autres adjuvants pharmaceutiques.

3. Procédé de préparation d'un médicament anticancéreux, caractérisé en ce que des composés de formule I dans laquelle les symboles chimiques $R_1$ à $R_6$, Alk, X, ainsi que la partie structurale A–B ont les significations indiquées dans la revendication 1, ou des sels de ces composés avec des acides physiologiquement inoffensifs sont mélangés ou homogénéisés avec des excipients et/ou des diluants ou des adjuvants usuels à des températures comprises entre 20 et 100°C, le mélange ainsi obtenu est versé dans des cellules creuses de dimensions appropriées, chargé dans des capsules de taille appropriée ou granulé puis pressé en comprimés avec addition éventuelle d'autres adjuvants usuels, pour la confection de préparations qui contiennent, dans l'unité posologique, de 10 à 500 mg de substance active de formule I.

4. Procédé de préparation d'un médicament à activité anticancéreuse, caractérisé en ce que des composés de formule I dans laquelle les symboles chimiques $R_1$ à $R_6$, Alk, X, ainsi que la partie structurale A–B ont les significations indiquées dans la revendication 1, ou des sels de ces composés avec des acides physiologiquement inoffensifs sont mélangés avec l'une ou plusieurs des substances suivantes: amidon, cellulose, lactose, formaline-caséine, amidon modifié, stéarate de magnésium, hydrogénophosphate de calcium, acide silicique fortement dispersé, talc, le mélange obtenu est granulé, éventuellement avec une solution aqueuse qui contient, en tant que constituant, l'un au moins des adjuvants gélatine, amidon, polysorbate, copolymère vinylpyrrolidone/acétate de vinyle et/ou mono-oléate de polyoxyéthylène sorbitanne, le granulat est éventuellement homogénéisé avec l'un ou plusieurs des adjuvants précités, et ce mélange est pressé en comprimés qui contiennent, dans l'unité posologique, de 10 à 500 mg de substance active de formule I.

5. Procédé de préparation d'un médicament à activité anticancéreuse, caractérisé en ce que des composés de formule I ou leurs sels avec des acides physiologiquement inoffensifs sont mis en suspension et homogénéisés, après addition de lécithine de soja, à des températures comprises entre 33 et 37°C, dans une graisse dure fondue, puis le mélange est versé dans des cellules creuses, l'unité posologique contenant de 10 à 500 mg de substance active.

6. Procédé de préparation d'un médicament à activité anticancéreuse, caractérisé en ce que des composés de formule I ou leurs sels avec des acides physiologiquement inoffensifs sont dissous dans une huile végétale à des températures comprises entre 30 et 100°C et la solution ainsi obtenue est additionnée d'une quantité suffisante d'huile végétale pour que la solution finale contienne de 0,5 à 2% en poids de substance active de formule I.

7. Utilisation de composés de formule générale I, dans laquelle $R_1$ représente un atome d'hydrogène, un groupement hydroxy ou un groupement alcanoyloxy en $C_2$–$C_6$, $R_2$ représente un atome d'hydrogène ou un atome d'halogène, $R_3$ représente un atome d'hydrogène ou un groupement alkyle en $C_1$–$C_6$, R4 représente un groupement hydroxy ou un groupement alcanoyloxy en $C_2$–$C_6$, $R_5$ représente un atome d'hydrogène ou un atome d'halogène et $R_6$ représente un atome d'hydrogène ou un atome d'halogène, Alk représente une chaîne alkylène en $C_2$–$C_{10}$, 4 groupements $CH_2$ voisins pouvant aussi être remplacés par un noyau 1,4-phénylène, A–B représente le groupement $HN–CH_2–CH_2–NH_2$, $H_2N–CH_2–CH–CH_2–NH_2$ ou

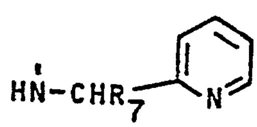

$R_7$ étant un atome d'hydrogène, un groupement alkyle en $C_1$–$C_6$ ou un reste phényle, et X est mis pour un équivalent d'un anion acceptable physiologiquement, pour la préparation de médicaments.